Europäisches Patentamt

European Patent Office

Office européen des brevets

(19)

(11) Publication number: **0 107 729**
**B1**

# EUROPEAN PATENT SPECIFICATION

(12)

(45) Date of publication of patent specification: **09.03.88**

(51) Int. Cl.⁴: **A 41 B 13/02**

(21) Application number: **83901999.9**

(22) Date of filing: **05.05.83**

(86) International application number:
**PCT/US83/00663**

(87) International publication number:
**WO 83/03754 10.11.83 Gazette 83/26**

(54) **ADJUSTABLE DIAPER WITH A BACKBAND AND FASTENING PROTECTION MEANS.**

(30) Priority: **05.05.82 US 375231**

(43) Date of publication of application:
**09.05.84 Bulletin 84/19**

(45) Publication of the grant of the patent:
**09.03.88 Bulletin 88/10**

(84) Designated Contracting States:
**AT BE CH DE FR GB LI LU NL SE**

(56) References cited:
**DE-A-2 906 655**
**GB-A-2 074 011**
**US-A-2 810 205**
**US-A-3 141 461**
**US-A-3 543 977**
**US-A-3 653 381**
**US-A-3 798 781**
**US-A-3 955 575**

(73) Proprietor: **COATES, Fredrica Vaughan**
**1608 Dublin Road**
**Charlottesville, VA 22903 (US)**

(72) Inventor: **COATES, Fredrica Vaughan**
**1608 Dublin Road**
**Charlottesville, VA 22903 (US)**

(74) Representative: **Patentanwälte Kohler -**
**Schwindling - Späth**
**Hohentwielstrasse 41**
**D-7000 Stuttgart 1 (DE)**

Courier Press, Leamington Spa, England.

## Description

This invention relates to a diaper, comprising:
a) a piece of material;
b) an attachment tab secured to one portion of the material and carrying a plurality of resiliently deformable, hook-like projections;
c) fastening means secured to another portion of the material and carrying a plurality of resiliently deformable, loop-like projections to be coupled to the hook-like projections of the attachment tab; and
d) protective cover means carrying loop-like projections and being arranged to couple with the hook-like projections of the attachment tab, thereby insulating the hook-like projections of the first attachment strip during washing.

US—A—3 955 575 discloses a diaper having fastening means which include hook-bearing attachment tabs secured to the edges of the piece of material forming the diaper. On the surface of the diaper opposite to the position where the hook-bearing types are attached, additional patches of loop-bearing pieces are fastened. When washing or replacing the diaper, the hook-bearing pieces may be folded over and fixed to these loop pieces so that the hook pieces cannot engage with other articles or tend to injure the skin.

Although the loop-bearing pieces attached to the diaper are expressly provided for being engaged with the hook-like projections on the attachment tabs in order to prevent the hook-like projections from an engagement with other articles or from injuring the skin, the loop-bearing pieces attached to the diaper are of no use if they are not purposely engaged with the hook-bearing tabs. It must be assumed that in general the user will not bother to fold over and fix the hook-bearing tabs to the loop-bearing pieces so that the danger of injuring other articles or becoming injured by engagement with other articles persists.

From GB—A—2 074 011, a diaper is known which has fastening strips carrying hook-like projections attached in parallel to a first edge of the piece of fabric forming the diaper. These strips are intended to be engaged with strips carrying corresponding hook-like projections and being attached adjacent a second edge of said piece of fabric, the second edge being parallel to the first edge. In the same position with respect to the second edge, but on the opposite side of the piece of fabric, further strips are attached which carry loop-like projections. These strips are intended to be engaged by further strips carrying hook-like projections, which are attached to the diaper in the middle of the first mentioned strip carrying the loop-like projections, said last mentioned strips have loose ends so that the portions of the diaper having attachment strips on their two sides may be positioned between the attachment strips positioned near the first edge of the diaper, of which strips the one is attached over its whole length to the diaper, whereas the other has loose ends which may be brought into engagement with the other side of the diaper.

Although the strip having loose ends may be brought into engagement with the underlying strip fastened along its whole length to the diaper, since these strips have complementary hook-like end loop-like projections, respectively, the strip having loose ends is not intended to provide protective cover means for the strip attached along its whole length to the diaper, since the last mentioned strip carries loop-like projections which need no protection. Further, near the other edge of the diaper, strips carrying hook-like projections are attached which are not provided with any protective cover means at all. Thus, GB—A—2 074 011 does not disclose anything which would be related to means for providing protection against injuries due to an engagement with the hook-like projections of attachment strips.

Besides of the danger that the hook-like projections engage other materials during handling so that damages may occur in the engaged material and/or in the hook-like projections themselves, the hook-like projections have the tendency to collect lint during washing which tends to deteriorate the adhesive effect of the diaper tabs and thus the diaper efficiency. Therefore, it is an object of the invention to provide a means for preventing lint from collecting on the hook-like projections of the attachment tabs.

This object is met by the invention in that the protective cover means is attached to the attachment tab and said attachment tab and said cover means have only one of their ends joined together and to the material with the remaining ends of at least one of said tab and said cover means being free-floating such that the hook-type and the loop-type filamentary materials are maintained face to face, in juxtaposition and the loop-type material extending over the hook-type material, whereby the attachment tab and cover means tend to self-close and couple to each other.

Thus, in the diaper according to the invention, the attachment tabs carrying hook-like projections are each provided with protective cover means carrying loop-like projections which are arranged in such a way that they tend to automatically couple with and thus protect the attachment tab. Especially, the hook-like projections of these attachment tabs are automatically prevented from lint collection, and this protection is independent of the user's will and action.

In one embodiment of the invention, the attachment tab is secured to an edge of the material to project laterally and outwardly therefrom. Said protective cover means includes a strip of material which has approximately the same size as or a greater width than said attachment tab.

The tabs and protective strips can be operatively attached to the diaper by means of a separate backband strip which in its turn is attached to the diaper upper edge for reinforcement.

Still other objects and advantages of the present invention will become readily apparent to those skilled in this art from the following detailed description, wherein only preferred embodiments of the invention are shown, simply by way of illustration of the best mode contemplated for carrying out the invention. As will be realized, the invention is capable of modifications in various aspects, all without departing from the invention as defined in the claims. Accordingly, the drawings and description are to be regarded as illustrative in nature, and not as restrictive.

Figure 1 is a front view of the outer surface of the diaper in accordance with the present invention, showing the position of attachment tabs and fastening strips thereon;

Figure 2A is a sectional view taken through the line 2A—2A of Figure 1 showing reinforced attachment of the attachment tab to the diaper backband and one embodiment for mounting protective tab cover strips on the diaper;

Figure 2B is similar to Figure 2A showing another embodiment for mounting the protective strip to the diaper outside the backband;

Figure 3 is similar to Figure 2A showing yet another embodiment of the invention; and

Figure 4 is a partial plan view of yet a further embodiment of the protective strip.

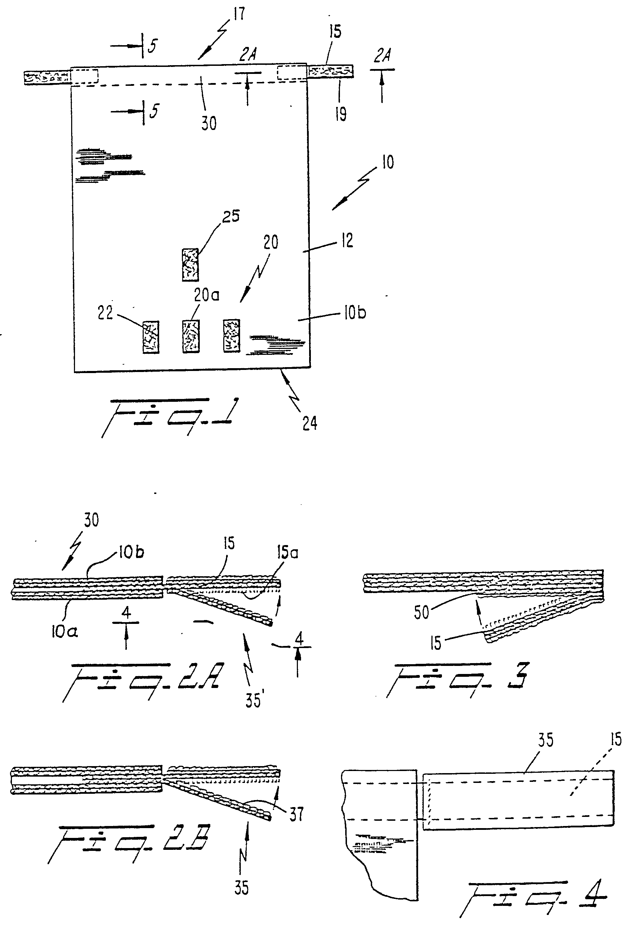

Referring to Figure 1, diaper 10 of the invention is shown in unfolded position to include a substantially rectangular, conventional diaper form 12, such as a two-ply layer of washable cotton fabric of the sponge center type. Diaper 10 includes inner and outer surfaces 10a, 10b respectively, provided on opposite sides of fabric 12 (see e.g. Figure 2A). Inner surface 10a is disposed adjacent the skin of an infant during wear. A backband 30 is provided along upper edge portion 17 of the diaper to provide additional strength for attachment of tabs 15 and greater moisture retention within the diaper.

To secure diaper 10 to the infant, a pair of attachment tab strips is respectively secured to upper adjacent corners of fabric 12 along the upper edge portion 17 of the diaper (see Figure 1). Tabs 15, as shown for example in Figure 2a, respectively include on one elongated rectangular surface thereof a plurality of resiliently deformable hook-like projections 15a defining an attachment surface 19. Each tab 15 projects laterally outward from upper edge 17 in unfastened position (Figure 1) with the surface 19 facing in the direction of inner surface 10a. The tabs 15 are preferably male Velcro® of the type available from Velcro® U.S.A. Inc., New York, or Aplix® tape, sold by Aplix® Inc., Pelham, New York.

To provide adjustable attachment of diaper 10 to accommodate any infant size, elongated fastening strips 20 and 25 are provided on outer surface 10b for mating engagement with tabs 15. Each strip 20 or 25 is of preferably rectangular shape and includes a plurality of loop-like projections 22 on the exposed surface thereof (Figure 1), such as female Velcro® tape or Aplix® tape, which provide excellent meshing engagement and gripping characteristics with hook projections 15a.

Strips 20 and 25 are preferably secured to outer surface 10b with through stitching and extend longitudinally along lower edge portion 24 of diaper 10, in spaced apart and parallel relationship to each other.

To prevent lint from collecting in the hook-like projections 15a of tabs 15 during repetitive washing and the like, protective cover means is provided. In one embodiment of the invention, shown in Figure 2B, the protective cover means includes a protective strip 35 of approximately the same size as tab 15. Protective strip 35 is preferably female Velcro® or Aplix® tape carrying loop-like projections 37. As shown in Figure 2B, each tab 15 overlaps with a strip 35 so that hook projections 15a and loop projections 37 face each other outside diaper form 12 in complete contacting registration. Corresponding ends of tabs 15 and strips 35 inserted within backband 30 are secured thereto with through stitching for improved reinforcement. Alternatively, as shown in Figure 2A, a protective strip 35' of shorter length than strip 35 is attached at one end thereof to tab 15 with a single line of through stitching outside upper edge portion 17 so that loop-like projections 37 and hook-like projections 15a face each other in the manner described above.

Protective strips 35 contact with attachment surface 19 of tabs 15 protects hook projections 15a during diaper washing and the like. Set up is made simply by gently pressing the protective strip 35 and the associated tab together prior to cleaning. Moreover, since the hooks 15a and loops 37 face each other on the overlapping strips biased together with the through stitching, a self-closing action is obtained with the above described embodiment. Further, by physically locating the protective strips 35 outside diaper form 12, the soiled diaper can be handled (e.g. toilet rinsing) without physically contacting the diaper form simply by engaging the tabs and protective strips. It will be further appreciated that strip 35 and tab 15 can be attached together to secure diaper 10 to a clothes line.

In an alternative embodiment to the invention, as shown in Figure 3, attachment tab 15 can be secured to protective strip 50 and to backband 30 by mounting the tab directly onto the strip along the outer corner side with a single line of through stitching. In this embodiment, the hook projections 15a are biased into covering position with the loop projections on strip 50 to achieve a self-closing action.

Figure 4 is an illustration of another improvement feature of the invention showing the use of the protective loop bearing cover strips discussed above being wider than the corresponding attachment tab 15. This feature ensures complete closure and protection of hook projections 15a from the external environment through full contacting registration.

From the foregoing, it can be seen that diaper 10 of the invention is capable of easy and reliable use on infants of any size, due to the features of and spacing between strips 20 and 25 in cooperation with tabs 15. Backband 30 provided along upper edge portion 17 provides for reinforced attachment conditions of tabs 15 and the protective strips, as described above, which is essential for prolonged daily use considering the constant opening and closing action both the tabs and the protective strips are required to undergo. In addition, the features of the invention set forth above are easily attachable to conventional diaper forms, resulting in cost effective modification of existing diapers and manufacture of new diapers with the above improvement features.

## Claims

1. A diaper, comprising:
a) a piece of material (12);
b) an attachment tab (15) secured to one portion of the material (12) and carrying a plurality of resiliently deformable, hook-like projections (15a);
c) fastening means (20) secured to another portion of the material (12) and carrying a plurality of resiliently deformable, loop-like projections (22) to be coupled to the hook-like projections (15a) of the attachment tab; and
d) protective cover means (35, 50) carrying loop-like projections (37) and being arranged to couple with the hook-like projections (15a) of the attachment tab (15), thereby insulating the hook-like projections of the first attachment strip during washing,
characterized in that the protective cover means (35, 50) is attached to the attachment tab (15) and said attachment tab (15) and said cover means (35) have only one of their ends joined together and to the material (12) with the remaining ends of at least one of said tab (15) and said cover means (35, 50) being free floating such that the hook-type and loop-type filamentary materials are maintained face to face, in juxtaposition and the loop-type material extending over the hook-type material, whereby the attachment tab and cover means tend to self-close and couple to each other.

2. A diaper according to claim 1, characterized in that said attachment tab (15) is secured to an edge of the material (12) to project laterally and outwardly therefrom; said protective cover means including a strip (35, 50) of material approximately the same size as the attachment tab.

3. A diaper according to claim 1 or 2, characterized in that said cover means (50) is positioned substantially entirely on said fabric.

4. A diaper according to claim 1 or 2, characterized in that said tab (15) and said cover means (35) extend outward from one side of said fabric.

5. A diaper according to claim 1 or 2, characterized in that said cover means (35) is under said tab (15).

6. A diaper according to any one of claims 1 to 5, characterized in that said protective cover means (35) has a width greater than a width of said tab (15).

7. A diaper to any one of claims 1 to 6, characterized in that
a) the piece of material (12) comprises a substantially rectangular piece of absorbent washable fabric (12) having opposite inner and outer surfaces (10a, 10b), said inner surface (10a) positionable to contact a wearer's skin to be diapered;
b) there is a pair of said attachment tabs (15) attached to an upper edge portion (17) of said fabric (12);
c) the fastening means (20) is secured to the outer surface (10b) of the fabric (12) adjacent a lower transverse edge (24) thereof for engaging the attachment tabs (15) to secure the fabric (12) as a diaper to the wearer; and
d) the protective cover means (35) is attached to the tabs (15) adjacent edges of the fabric (12).

## Patentansprüche

1. Windel, umfassend:
a) ein Stück Material (12);
b) eine Befestigungslasche (15), die an einem Abschnitt des Materials (12) befestigt ist und eine Vielzahl elastisch deformierbarer, hakenartiger Vorsprünge (15a) trägt;
c) Befestigungsmittel (20), die an einem anderen Abschnitt des Materials (12) befestigt sind und eine Vielzahl elastisch deformierbarer, schlingenartiger Vorsprünge (22) tragen, die mit den hakenartigen Vorsprüngen (15a) der Befestigungslasche (15a) in Eingriff zu bringen sind; und
d) schützende Abdeckmittel (35, 50), die schlingenartige Vorsprünge (37) tragen und so angeordnet sind, daß sie mit den hakenartigen Vorsprüngen (15a) der Befestigungslasche (15) in Eingriff kommen und dadurch die hakenartigen Vorsprünge (15) der ersten Befestigungslasche während des Waschens isolieren,
dadurch gekennzeichnet, daß die schützenden Abdeckmittel (35, 50) an der Befestigungslasche (15) angebracht sind und die Befestigungslasche (15) und die Abdeckmittel (35) nur an einem ihrer Enden miteinander und mit dem Material (12) verbunden sind und die anderen Enden wenigstens der Befestigungslasche (15) oder der Abdeckmittel (35, 50) frei beweglich sind, so daß die hakenartigen und die schlingenartigen fadenförmigen Materialien einander gegenüberliegend gehalten werden, sich überdecken und sich das schlingenartige Material über das hakenartige Material erstreckt, wodurch die Befestigungslasche und die Abdeckmittel die Tendenz haben, sich selbst zu schließen und miteinander in Eingriff zu kommen.

2. Windel nach Anspruch 1, dadurch gekennzeichnet, daß die Befestigungslasche (15) an einem Rand des Materials (12) befestigt ist, so daß sie seitlich nach außen davon absteht, und daß die schützenden Abdeckmittel einen Materialstreifen (35, 50) umfassen, der etwa die gleiche Größe hat wie die Befestigungslasche.

3. Windel nach Anspruch 1 oder 2, dadurch gekennzeichnet, daß die Abdeckmittel (50) im wesentlichen vollständig auf dem Stoff befestigt sind.

4. Windel nach Anspruch 1 oder 2, dadurch gekennzeichnet, daß die Lasche (15) und die genannten Abdeckmittel (35) sich von einer Seite des Stoffes nach außen erstrecken.

5. Windel nach Anspruch 1 oder 2, dadurch gekennzeichnet, daß sich die Abdeckmittel (35) unter der Lasche (15) befinden.

6. Windel nach einem beliebigen der Ansprüche 1 bis 5, dadurch gekennzeichnet, daß die schützenden Abdeckmittel (35) eine größere Breite hat als die Lasche (15).

7. Windel nach einem beliebigen der Ansprüche 1 bis 6, dadurch gekennzeichnet, daß

a) das Stück Material (12) aus einem im wesentlichen rechteckigen Stück eines absorbtionsfähigen waschbaren Stoffes (12) mit einander entgegengesetzten inneren und äußeren Flächen (10a, 10b) besteht, dessen innere Fläche (10a) in Kontakt mit der Haut des zu wickelnden Trägers gebracht werden kann;

b) ein Paar Befestigungslaschen (15) an einem oberen Randabschnitt (17) des genannten Stoffes (12) angebracht ist;

c) die Befestigungsmittel (20) an der Außenfläche (10b) des Stoffes neben dessen unterem Querrand (24) angebracht sind, um mit den Befestigungslaschen (15) in Eingriff gebracht zu werden, wenn der Stoff als Windel am Körper des Trägers befestigt wird; und

d) die schützenden Abdeckmittel (35) an den Laschen (15) neben den Rändern des Stoffes (12) angebracht sind.

## Revendications

1. Couche, comprenant:

(a) une pièce de matière (12);

(b) une patte d'attache (15) fixée à une partie de la matière (12) et portant une série de saillies en forme de crochets (15a), déformables élastiquement;

(c) des moyens de fixation (20) attachés à une autre partie de la matière (12) et portant une série de saillies en forme de boucles (22), déformables élastiquement, destinées à s'accrocher aux saillies en forme de crochets (15a) de la patte d'attache; et

(d) un système de recouvrement protecteur (35, 50) portant des saillies en forme de boucles (37) et agencé pour coopérer avec les saillies en crochets (15a) de le patte d'attache (15), de manière à isoler ainsi les saillies en crochets de la première patte d'attache durant le lavage,

caractérisée en ce que le système de recouvrement protecteur (35, 50) est attaché à la patte d'attache (15) et en ce que cette patte d'attache (15) et ledit système de recouvrement (35) ne sont réunis eux et à la matière (12) que par une seule de leurs extrémités, les extrémités restantes d'au moins l'un des éléments formés par cette patte (15) et le système de recouvrement (35, 50) étant libres de telle sorte que les matières filamentaires du type crochets et du type boucles sont maintenues face à face en juxtaposition et que la matière du type à boucles s'étend par-dessus la matière du type à crochets, la patte d'attache et le système de recouvrement tendant à se refermer l'un sur l'autre et à s'accoupler.

2. Couche suivant la revendication 1, caractérisée en ce que la patte d'attache (15) est fixée à un bord de la matière (12) de manière à faire saillie latéralement et vers l'extérieur depuis celle-ci, le système de recouvrement protecteur comprenant une languette (35, 50) faite d'une matière ayant approximativement les mêmes dimensions que la patte d'attache.

3. Couche suivant la revendication 1 ou 2, caractérisée en ce que le système de recouvrement (50) et disposé pratiquement totalement sur le tissu susdit.

4. Couche suivant la revendication 1 ou 2, caractérisée en ce que la patte (15) et le système de recouvrement (35) s'étendent vers l'extérieur depuis un côté du tissu susdit.

5. Couche suivant la revendication 1 ou 2, caractérisée en ce que le système de recouvrement (35) se trouve sous la patte (15).

6. Couche suivant l'une quelconque des revendications 1 à 5, caractérisée en ce que le système de recouvrement protecteur (35) a une largeur supérieure à celle de la patte (15).

7. Couche suivant l'une quelconque des revendications 1 à 6, caractérisée en ce que:

(a) la pièce de matière (12) consiste en une pièce essentiellement rectangulaire faite d'un tissu lavable absorbant (12), présentant des surfaces interne et externe opposées (10a, 10b), la surface interne (10a) pouvant être disposée pour entrer en contact avec la peau d'un porteur à langer;

(b) deux pattes d'attache (15) sont attachées à une partie marginale supérieure (15) du tissu susdit (12);

(c) le système de fixation (20) est attaché à la surface extérieure (10b) du tissu (12) au voisinage d'un bord transversal inférieur (24) de celui-ci, pour coopérer avec les pattes d'attache (15) en vue de la fixation du tissu (12) à titre de couche sur l'usager; et

(d) le système de recouvrement protecteur (35) est attaché aux pattes (15) au voisinage des bords du tissu (12).

Fig. 1

Fig. 2A

Fig. 3

Fig. 2B

Fig. 4